# EUROPEAN PATENT APPLICATION

(11) **EP 1 739 089 A1**
(43) Date of publication of application: **03.01.2007**
(21) Application number: 04821886.1
(22) Date of filing: 08.11.2004
(51) Int. Cl.: C07K 1/18

(54) **METHOD OF SEPARATING PROTEIN**

(30) Priority: 30.03.2004 JP 2004098022
(71) Applicant: Yanagisawa, Hiroshi, Midori-ku, Nagoya-shi Aichi 4580804 (JP); Sakakibara, Yoko, Taketoyo-cho, Chita-gun Aichi 4702337 (JP)
(72) Inventor: Yanagisawa, Hiroshi, Midori-ku, Nagoya-shi Aichi 4580804 (JP); Sakakibara, Yoko, Taketoyo-cho, Chita-gun Aichi 4702337 (JP)
(74) Representative: Müller Fottner Steinecke
(86) International application number: PCT/JP2004/016549
(87) International publication number: WO 2005/100379

(57) **Abstract**

It is intended to provide a method whereby a protein can be separated and purified at a high accuracy by a convenient procedure. A sample containing the desired protein is brought into contact with an ion exchanger under first conditions at a high ionic strength and at p H value not in the vicinity of the isoelectric point of the desired protein. Next, the component adsorbed by the ion exchanger is eluted under second conditions at a lower ionic strength than in the first conditions and at a pH value closer to the isoelectric point of the desired protein than in the first conditions.

## Description

### Technical Field

The invention relates to a method of separating proteins taking advantage of their isoelectric points and applications of the method.

### Background of the Invention

Salting-out, gel filtration (molecular sieve) and various chromatographic techniques taking advantage of physical and chemical properties of the substance to be purified have been usually combined for separating and purifying proteins and glycoproteins, and affinity chromatography using lectins has been particularly considered to be effective for purifying glycoproteins.

### Disclosure of Invention

### Problems to be solved by the invention

However, the yield of the substance to be extracted may be lowered or deterioration of the extracted substance may occur depending on the separation and purification method, and the procedure of the method may be complicated.
In addition, lectins should be selected for respective types of the sugar chain of the glycoprotein in advance in the affinity chromatography that has been thought to be effective for purification of the glycoprotein, and discrimination of the type of the sugar chain of the glycoprotein to be separated is inevitable. It is also a defect of this method that the cost for separation and purification is expensive.
The objective of the invention is to solve at least one of the above-mentioned problems.

### Means for solving the problem

In view of the above-mentioned objective, the inventors of the invention have paid attention to isoelectric point chromatography that has been used for a protein purification method. Isoelectric point chromatography takes advantage of isoelectric points of proteins comprising the steps of adsorbing a target protein on a support, and eluting the target protein. It has been a common method in conventional isoelectric point chromatography to adsorb the protein on the support at a low ion strength, and elute the protein by increasing the ion strength. However, although it is highly possible that a target substance can be adsorbed as the ion strength is lower, it is also possible that substances other than the target substance are also adsorbed. The inventors of the invention have paid attention to the above-mentioned problems, and have made intensive studies on the conditions for separating the glycoprotein. As a result, it was found that the glycoprotein in the sample could be selectively separated and recovered under conditions contradictory to conventional common knowledge, namely the adsorption is performed at high ion strength, and elution is performed at low ion strength. It was confirmed from further studies that glycoproteins having weak binding ability to the support could be separated and recovered by lowering the ion strength for adsorption. While the separation method having the above-mentioned features may be advantageous for the separation and recovery of the glycoprotein, it was also suggested that the method may be also applicable to simple and efficient separation and purification of ordinary proteins, as well as the glycoprotein, depending on the adsorption conditions and supports to be used.
The invention has been completed based on the above-mentioned results and provides the following features.
(1) A method for separating proteins comprising the steps of:
   (a) adsorbing a sample containing a target protein on an ion exchanger by allowing the sample to contact the ion exchanger under a first condition at high ion strength and at a pH outside of the vicinity of an isoelectric point of the target protein; and
   (b) eluting the component adsorbed on the ion exchanger under a second condition at lower ion strength than in the first condition, and at a pH closer to the isoelectric point side than in the first condition.
(2) The method for separating proteins according to (1), wherein the first condition comprises using a buffer solution with a concentration of 0.05 M or more.
(3) The method for separating proteins according to (1) or (2), wherein the first condition comprises using a high concentration of a buffer solution comprising a combination of a weak acid and a weak base.
(4) A method for separating proteins comprising the steps of:
   (a) adsorbing a target protein on an ion exchanger by allowing the sample containing the target protein to contact the ion exchanger under a first condition at first ion strength and at a pH outside of the vicinity of an isoelectric point of the target protein; and
   (b) eluting the component adsorbed on the ion exchanger under a second condition at an ion strength lower than or equal to the ion strength in the first condition, and at a pH closer to the isoelectric point than in the first condition.
(5) The method for separating proteins according to any one of (1) to (4) comprising the following step between step (a) and step (b):
   (c) washing the ion exchanger under a condition not eluting the target protein adsorbed on the ion exchanger.
(6) The method for separating proteins according to (5), wherein step (c) is applied under substantially the same condition as the first condition.
(7) The method for separating proteins according to any one of (1) to (6), wherein
   the pH in the first condition is lower than the isoelectric point of the target protein,
   the ion exchanger is a cation exchanger, and
   the pH in the second condition is in the vicinity of or higher than the isoelectric point of the target protein.
(8) The method for separating proteins according to any one of (1) to (6), wherein
   the pH in the first condition is higher than the isoelectric point of the target protein,
   the ion exchanger is an anion exchanger, and
   the pH in the second condition is in the vicinity of or lower than the isoelectric point of the target protein.
(9) The method for separating proteins according to any one of (1) to (8), wherein the first condition comprises using a tris-succinate buffer solution.
(10) The method for separating proteins according to any one of (1) to (9) using, in the second condition, a buffer solution comprising a combination of the same acid and the same base as used in the first condition.
(11) The method for separating proteins according to any one of (1) to (10) using, in the second condition, a buffer solution having a pH in the vicinity of isoelectric point of the target protein.
(12) The method for separating proteins according to any one of (1) to (11), wherein
   the sample contains a plurality of target proteins, and
   step (b) comprises the step of continuously eluting the proteins under a solvent condition corresponding to the isoelectric points of respective target proteins.
(13) The method for separating proteins according to any one of (1) to (12), wherein the protein is a glycoprotein.

### Effect of the Invention

Adsorption of unnecessary substances to a support (ion exchanger) can be efficiently prevented in the method according to the invention since adsorption(and washing) is performed under a condition at high ion strength. A condition for lowering the ion strength for elution is effective for specifically eluting the target protein by preventing elution of the unnecessary substances due to increased ion strength. According to the invention employing such condition, it is possible to separate and purify the target protein by a simple operation within a short period of time.

### Brief Description of the Drawings

Fig. 1 shows the result (chromatogram) of purification of pea diamine oxidase by the method according to the invention.
Fig. 2 is a table showing the yield of diamine oxidase purified by the method of the invention. The lower table shows the yield when diamine oxidase is purified by a conventional method (Purification and Characterization of Amine Oxidase from Pea Seedlings, F. Vianello, A. Malek-Mirzayans, M. Luisa Di Paolo, R. Stevanata, and A Rigo, Protein Express and Purification 15, 196-201(1999)).
Fig. 3 shows the result (chromatogram) of purification of egg white by the method according to the invention.
Fig. 4 shows the result of electrophoresis of the final product of egg white purified by the method according to the invention.
Fig. 5 shows the result (chromatogram) of egg white purified by the method according to the invention.
Fig. 6 shows the result of electrophoresis of the final product of egg white purified by the method according to the invention.
Fig. 7 shows the results (chromatograms) of separation of egg white albumin, transferrin and streptavidin by the method according to the invention using CM Sephadex as an ion exchanger.
Fig. 8 shows the results (chromatograms) of separation of egg white albumin, transferrin and streptavidin by the method according to the invention using CM Sepharose as an ion exchanger.
Fig. 9 shows the results (chromatograms) of separation of egg white albumin, transferrin and streptavidin by the method according to the invention using SP Sephadex as an ion exchanger.
Fig. 10 shows the results (chromatograms) of separation of egg white albumin, transferrin and streptavidin by the method according to the invention using SP Sepharose as an ion exchanger.
Fig. 11 shows the result of chromatography adsorbed under a condition of higher ion strength (0.2 M tris-succinate buffer).
Fig, 12 shows the result of chromatography washed with a high concentration of a buffer solution.
Fig. 13 shows a result of separation (chromatogram) of carbonic anhydrase and peroxidase by the method according to the invention.
Fig. 14 shows the result (chromatogram) of separation of all the proteins loaded on the column using the tris-acetic acid buffer solution.
Fig. 15 shows the result (chromatogram) of separation of all the proteins loaded on the column using the tris-citric acid buffer solution.

### Best Mode for Carrying Out the Invention

The invention relates to a method for separating proteins, and comprised following steps (a) and (b) in the first aspect:
(a) adsorbing a sample containing a target protein on an ion exchanger by allowing the sample to contact the ion exchanger under a first condition at high ion strength, and at a pH outside of the vicinity of an isoelectric point of the target protein; and
(b) eluting the component adsorbed on the ion exchanger under a second condition at an ion strength lower the ion strength in the first condition, and at a pH closer to the isoelectric point side than in the first condition.
   The protein as a target of separation (also referred to the "target protein" in the specification) is not particularly limited to in the invention. The target is preferably a protein having sugar chains, or a glycoprotein. The term "protein" in the specification represents a concept including the glycoprotein, except that the term is clearly described in contrast to the glycoprotein.
   Specific examples of the proteins as the target of separation in the invention include egg white albumin, diamine oxidase and transferrin. According to the invention as shown in examples below, separation was successful in all the attempts of separation of egg white albumin, diamine oxidase and transferrin. Accordingly, the invention is applicable for a variety of glycoproteins.

While the term "separation" as used in the invention refers to separation of the target protein from a sample, the term is also used to include a concept of "purification" herein.
For example, the invention may be used for separating only the target protein from a sample containing plural kinds of proteins (including a case where a part of them is glycoproteins). The plural kinds of proteins may be separated by one operation. The plural kinds of the proteins serve as the target proteins in this case. The invention is particularly favorable for separating specified proteins or glycoproteins in the sample containing proteins and glycoproteins together. Specifically, when proteins and glycoproteins having sugar chains added thereto are present together in the sample, the invention may be utilized for separating either of them.

Step (a) is performed under a condition (first condition) at high ion strength and at a pH outside of the vicinity of the isoelectric point of the target protein. The sample contacts an ion exchanger typically by adding the sample to a column packed with the ion exchanger in this step. The sample is usually added to the column equilibrated with an appropriate buffer solution in advance. A continuous operation from adsorption to elution may be facilitated by employing such mode of column chromatography. On the other hand, the sample may be directly added to the ion exchanger not packed in a specified vessel such as a column in order to permit the target protein to contact the ion exchanger. Such batch type treatment method is advantageous for facilitating a large quantity of the sample to be treated in one operation.

Step (a) is performed at high ion strength. It was revealed by the inventors that adsorption at high ion strength prevents foreign substances from being adsorbed, or the target protein in the sample can be specifically adsorbed. Proteins were adsorbed at low ion strength by using, for example, a buffer solution with a concentration of 20 mM in conventional isoelectric point chromatography. However, one feature of the invention is that proteins are adsorbed at relatively higher ion strength than conventional conditions by using, for example, a tris-succinate buffer solution with a concentration of as high as 0.1 M, instead of adsorbing at above-mentioned low ion strength. The term "high ion strength" as used in the invention refers to, for example, a concentration of about 0.05 M or more, preferably about 0.075 M or more, more preferably about 0.1 M or more, and further preferably about 0.2 M or more when a buffer solution comprising a combination of a weak acid and weak base is used, although the concentration differs depending on the kind of the target protein and the kind of the sample containing the protein, and on the kind of the buffer solution used. The upper limit is not particularly limited to, and a buffer solution with a concentration in the range from about 0.005 M to about 1.5 M, from about 0.075 M to about 1.0 M, or from about 0.1 M to about 0.5 M can be employed.

The ion exchanger is selected by taking into consideration the isoelectric point of the protein (target protein) to be separated. The target protein is adsorbed under a condition for allowing the target protein to be positively charged, or at a lower pH than the isoelectric point of the target protein (low pH condition), for ensuring adsorption of the target protein on the ion exchanger when a cation exchanger is used. The low pH condition differs depending on the isoelectric point of the target protein, and the pH may be adjusted in the range from 4.0 to 4.3, for example, when the isoelectric point of the target protein is at pH 4.5. When the sample contains plural target proteins and these proteins are to be continuously separated or recovered, the pH is adjusted so that all the proteins are positively charged. For example, when the target proteins having isoelectric points at pH 4.2, 4.5 and 6.2, respectively, are mixed together, the proteins may be adsorbed, for example, in the pH range from 4.0 to 4.1.
On the contrary, the protein is adsorbed under a condition for allowing the protein to be negatively charged, or at a higher pH (low pH condition) than the isoelectric point of the target protein when an anion exchanger is used.
The target protein can be adsorbed on the ion exchanger as described above by selecting a condition in which the pH is not in the vicinity of the isoelectric point of the target protein.

It is preferable for determining the pH for adsorption to take pH stability of the target protein into consideration. In other words, the pH is preferably controlled in the range not deactivating the protein or decreasing the activity of the protein, when the target protein is required to be separated while it maintains the activity.

The kind of the ion exchanger is not particularly limited to. Examples of the ion exchanger (resin) available include styrene, acrylic, methacrylic, phenol, aliphatic, pyridine, dextran or cellulose base reins into which cationic, anionic or amphoteric ion exchange groups are introduced. For example, carboxylic groups (CM) or sulfuric groups (SM and Mono S) are used as the cationic ion exchange group, while amino groups (DEAE, QAE and Mono Q) are used as the anionic ion exchange groups. A variety of ion exchangers are commercially available (for example those sold by Seikagaku Co., Amersham Biosciences K.K.), and commercially available ion exchangers providing appropriate characteristics may be used in the invention.

The buffer solution used for allowing the sample to adsorb preferably comprises a combination of a weak acid and weak base (such as tris-succinate buffer solution, tris-citric acid buffer solution and tris-acetic acid buffer solution). Use of the buffer solution having such characteristics permits a buffer solution used in the elution condition (second condition) described below to be prepared by decreasing the concentration of the acid or base constituting the buffer solution or, in other words, a buffer solution having low ion strength and at a pH closer to the isoelectric point side of the target protein than in the first condition can be prepared. Accordingly, there is no need to prepare plural kinds of buffer solutions having different combinations of the acid and base. The entire operation is further simplified since shift from adsorption (and washing) to elution is possible by merely changing the proportion of the acid or base in the buffer solution, and a chain of continuous operations is possible.
When the ion exchanger is equilibrated in advance to adsorption, the same buffer solution as used in adsorption may be usually used for equilibration.

An elution step (step b) follows step a. The elution step is performed under the second condition at a lower ion strength than in the first condition, and at a pH closer to the isoelectric point side of the target protein than in the first condition. Since the method of the invention is featured in that the ion strength in the second condition is adjusted to be lower than the ion strength in the first condition and the protein is adsorbed at a high ion strength and eluted at a low ion strength, the method is effective for specifically separating the target protein (in particular glycoprotein). In other words, lowering the ion strength for elution prevents unnecessary components adsorbed on the ion exchanger from being eluted, or the target protein can be selectively and efficiently eluted.
The ion strength in the second condition may be determined by taking other conditions (such as the kind of the target protein, the kind of the sample containing the protein, or the kind of the buffer solution) into consideration, with the proviso that it is essential that the ion strength is lower than the ion strength in the first condition. For example, when a buffer solution comprising a weak acid and weak base is used, the second condition is determined so that the concentration of the weak acid or base is lower than the concentration in the first condition within the range from about 0.05 M to about 0.5 M, preferably from about 0.075 M to about 0.1 M, and further preferably from about 0.1 M to about 0.75 M.

Subsequently, the pH in the second condition is adjusted to be closer to the isoelectric point side of the target protein than in the first condition. Specifically, when the protein is adsorbed in the adsorption step using a cation exchanger as the ion exchanger, the pH in the second condition is adjusted to be in the vicinity of or higher than the isoelectric point of the target protein. When the protein is adsorbed in the adsorption step using an anion exchanger as the ion exchanger, on the other hand, the pH in the second condition is adjusted to be in the vicinity of or lower than the isoelectric point of the target protein.

An elution solution satisfying the above-mentioned second condition is prepared in the elution step, and the ion exchanger after the adsorption step is made to contact the elution solution. When the sample subjected to the adsorption step contains plural kinds of the target proteins, these plural proteins can be continuously eluted by continuous elution at solvent conditions corresponding to respective isoelectric points of the target proteins.

While a buffer solution having a quite different composition from the buffer solution used in the adsorption step may be used, it is preferable to use a buffer solution obtained by reducing the concentration of either the acid or the base in the buffer solution used in the adsorption step (the other concentration is preferably maintained as before). A buffer solution comprising a combination of the same acid and the same base as in the buffer solution used in the adsorption step is used in the elution step in the above-mentioned aspect. In other words, one of the features of the second condition, or a low ion strength, as well as the other feature, or a pH closer to the isoelectric point side of the target protein than in the first condition, may be simultaneously satisfied in this aspect. When the pH of the buffer solution used in the adsorption step is lower than the isoelectric point of the target protein, the ion strength is lowered by reducing the concentration of the acid constituting the buffer solution. In addition, the pH shifts to a pH closer to the isoelectric point side of the target protein (shift to higher pH), and the second condition is satisfied. Likewise, when the pH of the buffer solution used in the adsorption step is higher than the isoelectric point of the target protein, the ion strength is lowered by reducing the concentration of the base constituting the buffer solution. In addition, the pH shifts to a pH closer to the isoelectric point side of the target protein (shift to lower pH), and the second condition is satisfied. According to the above-mentioned aspects (the aspect for permitting the concentrations of the acid or base to change in the buffer solution used), reduction of the ion strength and adjustment of the pH may be simultaneously achieved by simple operations. These aspects are particularly effective when a plurality of proteins or glycoproteins having different isoelectric points is present in the sample, and when each of these proteins are to be separated or fractionated, since continuous reduction of the ion strength and control of the pH are also possible by simple operations.

A washing step (step c) is preferably interposed between the adsorption step (step a) and elution step (step b). The object of the washing step is to remove unnecessary components (components other than the target protein in the sample) adsorbed on the ion exchanger after the adsorption step by washing. Accordingly, the washing solution used preferably satisfies a condition by which the unnecessary components are effectively removed while the target protein adsorbed on the ion exchanger is not eluted. For example, the ion exchanger is washed in the washing step with a buffer solution having the same composition as the buffer solution used in the adsorption step (or substantially under the same condition). It is needless to say that the composition of the buffer solution may be slightly changed for enhancing the effect of washing. A buffer solution to which a small amount of appropriate salts are added may be used as the washing solution. It is preferable to wash the ion exchanger with a sufficient amount of the washing solution in order to attain a good washing effect. For example, when the ion exchanger packed in a column is used, the column is washed using the washing solution twice to 30 timed by volume, preferably 3 to 20 times by volume, of the volume of the ion exchanger.

While the temperature is not particularly limited to in the method of the invention, the temperatures of a series of operations are usually room temperature or a low temperature (for example in the range from 4 to 10°C).

Other features of the invention will be described below. Descriptions corresponding to those in the above-mentioned aspects that are not particularly mentioned hereinafter and details thereof omitted in the descriptions - such as the contact method between the sample and ion exchanger, the kind of the ion exchanger and corresponding adsorption conditions, selection of pH for adsorption, selection of pH for elution, elution methods and preference of concomitant use of the washing step - are quoted by way of references in the descriptions below.
The separation method of this aspect comprises following steps (a) and (b):
(a) permitting the sample containing the target protein to contact the ion exchanger under a first condition at a first ion strength and at a pH not in the vicinity of the isoelectric point of the target protein; and
(b) eluting the component adsorbed on the ion exchanger under a second condition at an ion strength equal to or lower than the ion strength in the first condition at a pH closer to the isoelectric point side than in the first condition.

Step (a) is performed under a condition (first condition) at the first ion strength and at a pH outside of the vicinity of the isoelectric point of the target protein. A relatively low ion strength is employed as the first ion strength, since this condition affords a favorable condition for permitting a protein (or glycoprotein) having weak adsorption ability to the support to be adsorbed. Accordingly, the separation method in this aspect is usually utilized for separation and recovery of the protein (or glycoprotein) having weak adsorption ability to the support.

While the "first ion strength" of the invention usually varies depending on the kind of the target protein, the kind of the sample containing the protein or the kind of the buffer solution used, the concentration is, for example, about 0.08 M or less, preferably about 0.05 M or less, more preferably about 0.03 M or less and further preferably about 0.02 M or less when a buffer solution comprising a weak acid and weak base is used. The lower limit is not particularly limited to, and a buffer solution with a concentration in the range from about 0.001 M to about 0.08 M, from about 0.01 M to about 0.05 M or from about 0.02 M to about 0.03 M may be used.

The elution step (step b) follows the step (a). This elution step is preformed under the second condition at an ion strength of equal to or lower than the ion strength in the first condition, and at a pH closer to the isoelectric point of the target protein than in the first condition. The ion strength in the second condition is determined to be equal or lower than the ion strength in the first condition. The method of the invention is featured in that a higher ion strength is not used for elution, and is effective for specifically separating the target protein (particularly the glycoprotein). Selecting a low ion strength for elution permits unnecessary components adsorbed on the ion exchanger to be prevented from being eluted. In other words, the target protein can be specifically and efficiently eluted.
The ion strength in the second condition may be determined by taking other conditions (the kind of the target protein, the kind of the sample containing the protein, or the kind of the buffer solution used) into consideration under an essential condition that the ion strength in the second condition is equal to or lower than the ion strength in the first condition.
The phrase "the same ion strength" refers to a fact that two levels of the ion strength to be compared are the same, or the elution patterns eluted with the two levels of the compared ion strength are only slightly different to an extent that no substantial difference is recognized. In a specific example of the "equal ion strength", the protein is eluted at an ion strength about 1 to 5 times of the ion strength for adsorption (for example, the concentration of the buffer solution is about 0.02 M for adsorption and about 0.02 to about 0.1 M for elution). The protein may be eluted at an ion strength preferably about 1 to 3 times, more preferably about 1 to 1.5 times, and most preferably about 1 to 1.2 time of the ion strength for adsorption.
When a buffer solution comprising a weak acid and a weak base is used with a concentration of the acid or base in the range from about 0.001M to about 0.08 M, from about 0.01 M to about 0.05 M, or from about 0.02 M to about 0.03 M, the ion strength in the second condition may be determined so as to be equal or lower than in the first condition.
Examples (including experimental examples) of the invention will be described below.

### [Example 1]

### <Simple purification method for diamine oxidase>

We attempted to develop a simple purification method employing separation based on the isoelectric point, which is used to purify a glycoprotein. We used diamine oxidase (hereinafter, referred to as "DAO") from Pea as a model glycoprotein. Namely, the separation of DAO was attempted according to the following procedures, and the results were evaluated.
a. Materials and methods
   SP-Sephadex c-50 (Amersham Biosciences K.K. 2.5 x 6 cm), a cation resin, was equilibrated with 0.1 M Tris succinate buffer, pH 6.5. Pea epicotyls were homogenized with 0.2 M Tris succinate buffer, pH 7.5. The homogenate was squeezed through a nylon mesh (74 µm) to remove insoluble substances. The solution was brought to pH 6.5 with saturated succinic acid and to twice the volume of the extraction buffer with distilled water, and then centrifuged at 15000 g for 20 min. (Step 1: crude extract) Next, the crude extract (the supernatant of centrifugation) was applied to the SP-Sephedex c-50 column. After the column was washed with 20 volumes of 0.1 M Tris succinate buffer, pH 6.5, the enzyme was eluted with 0.1M Tris succinate buffer, pH 8.0. The fractions were collected by a Model 2110 fraction collector (Bio-Rad Laboratories; 5.1 mL/tube).
b. Result
   Figure 1 shows the results of chromatography performed as described above. The horizontal axis is the fraction number (time axis) and the spindle axis is OD at 280nm (left), and DAO activity (right). The peaks for OD280 and DAO activity are in the vicinity of fraction number 5. Active fractions (3-11) were gathered and used as a purified enzyme (Step 2: Final purification). DAO activity, purification and yield are summarized in the Table in Fig. 2. The purified enzyme had a specific activity of 1.38µkat/mg protein with purification of 115 and this was almost the same as the value reported in the literature. The yield with this procedure (88.7%) is much greater than that with the previous method.
   To confirm purity, the purified enzyme was subjected to SDS electrophoresis. As a result, a single band was seen with silver staining (result not shown).
   When crude extract from pea epicotyls was applied to an SP-Sephadex column equilibrated with 0.1 M Tris succinate buffer, pH 6.5, and the column was then washed with the same buffer, most bulk proteins were not absorbed. Next, DAO was eluted with 0.1 M Tris succinate buffer, pH 8.0.
c. Evaluation
   Diamine oxidase (DAO) is a glycoprotein from Pea. McGuil et al. established a method for its purification in 1994. The method involved crude extract, ammonium sulfate precipitation, organic solvent precipitation, DEAE-cellulose column chromatography, gel filtration, and hydroxyapatite column chromatography. These are general purification techniques that gradually remove contaminating proteins through repeated precipitation and dialysis, and take advantage of the electric properties of proteins and differences in molecular weights. These techniques usually require 3-4 days.
   The present simple purification method, which includes cation exchange and Tris succinate buffer, enabled us to purify DAO to homogeneity by silver staining in 12 hours or less while simultaneously achieving a higher yield than the previous method.

### [Example 2]

### <Adsorption properties of glycoproteins>

### (1-1) Purification of ovalbumin 1 (with elution based on the isoelectric point)

To examine whether the simple purification method that was shown to be effective for purifying DAO could be applied to the purification of other glycoproteins, we clarified the adsorption properties of some resins in egg white which has been shown to contain 13 proteins and glycoproteins.
a. Materials and methods
   CM-Sephadex C-50 (Amersham Biosciences K.K. 1.6 x 6 cm), a cation resin, was equilibrated with 0.1 M Tris succinate buffer, pH 4.0.
   The volume of egg white was measured and five volumes of distilled water were added. This mixture was stirred for 30 min. To 20 ml of this solution was added 50 ml of 0.2 M Tris succinate buffer, pH 7.5, and the pH was adjusted to 4.0 with saturated succinic acid. In addition, this solution was brought to 100mL with distilled water. (Ovomucin can be efficiently removed from egg white by dilution with distilled water two or three times, or by precipitation at pH 4.0.) The solution was then centrifuged at 15000 g for 20 min, and 10mL of the supernatant was applied to the column. After the column was washed with 10 volumes of 0.1 M Tris succinate buffer, pH 4.5, and we confirmed that the OD of the eluate at 280 nm was below 0.1, the elution buffer was replaced by 0.1 M Tris succinate buffer, pH 8.0. The fractions were collected by a Model 2110 fraction collector (Bio-Rad Laboratories; 5.1 mL/tube).
b. Result
   Figure 3 shows the results of chromatography performed as described above. The horizontal axis is the fraction number (time axis), and the spindle axis is OD at 280nm. The peak of 280nm was located in fraction numbers 22-36. These fractions were gathered as the final purified enzyme. A single band was seen in SDS electrophoresis with silver staining. (Fig. 4). A similar result was seen upon purification with other cation-exchangers according to a similar procedure. (results not shown)
c. Evaluation
   Ovalbumin (isoelectric point pH4.5) could be efficiently collected from egg white in one step.

### (1-2)Purification of ovalbumin 2 (elution above the isoelectric point) The elution conditions were changed and ovalbumin was purified as in

### (1-1). Thus, 0.1 M Tris succinate buffer, pH 4.75, was passed through the column. In addition, 0.1M Tris was passed through the column until the OD at 280nm was 0.1 or less. The fractions were collected by a Model 2110 fraction collector (Bio-Rad Laboratories; 5.1 mL/tube).

Figure 5 shows the results of chromatography performed as described above. The horizontal axis is the fraction number (time axis) and the spindle axis is OD at 280nm. The peak of 280nm is in the vicinity of fraction numbers 14-19, which were gathered and used as a final purified protein. Figure 6 shows the results of protein silver staining by SDS-PAGE. The only band of ovalbumin was confirmed in fraction numbers 14-19. Upon washing with 0.1 M Tris (about pH 9.5), although absorption of OD280nm was noted in fraction numbers 32-35, a band of ovalbumin was not confirmed by SDS-PAGE.
The elution conditions and the results of purifications 1 and 2 of ovalbumin (isoelectric point pH4.5) are reference points for solving the problem of how to most efficiently collect a target protein.
The pH of the buffer in the ovalbumin-elution step is slightly different between purifications 1 and 2.
In purification 1, buffer with the same pH (4.5) as the isoelectric point of ovalbumin is used as the buffer for elution. In purification 2, the isoelectric point of ovalbumin (pH 4.5) is between the pH (4.75) of the buffer solution used for elution and the pH (4.0) of the buffer solution used for adsorption and washing.
Although the results with purification 1 show that ovalbumin is eluted when the pH of the elution buffer is equal to the isoelectric point of ovalbumin(Fig. 3), the results of purification 2 show that ovalbumin that has adsorbed to the cation-exchanger in adsorption & washing buffer (pH 4.0) is rapidly eluted by buffer solution (pH4.75) at a pH that exceeds the isoelectric point (Fig. 5). When elution is performed at the isoelectric point of ovalbumin (pH 4.5) (purification 1, Fig. 3), fraction numbers 22-36 (total 15) were gathered at an OD 280nm of about 0.5. When elution was performed at a pH greater than the isoelectric point of ovalbumin (pH 4.75; purification 2, Fig. 5), fraction numbers 14-19 (total 5) were gathered at an OD 280nm of about 1.2, and after fraction number 20 (division with pH4.75), there was little, if any, absorption at OD 280nm. These results suggest that more efficient collection may be possible when the pH of the elution buffer exceeds the isoelectric point of the target protein, as demonstrated purification 2 and the results of protein silver staining in SDS-PAGE (Fig. 6). However, when an adsorbed substance has an isoelectric point near that of the target protein, and this adsorbed substance shows weaker adsorption than the target protein, careful attention is required.

### (2) Application of the above purification procedure as an isolation method

Next, we examined whether the above simple purification method could be effective for isolating glycoproteins from a sample that contained two or more glycoproteins mixed together. Specifically, we examined whether or not each glycoprotein coud be isolated from a sample that contains two or more glycoproteins and proteins mixed together.
a. Materials and methods
   A solution that contained ovalbumin (isoelectric point pH4.5, molecular weight 45k, Sigma-Aldrich Corporation) and transferrin (isoelectric point pH6.2, molecular weight 80k, Sigma-Aldrich Corporation) as glycoproteins and streptavidin (isoelectric point of total protein pH6.4, isoelectric point of the surface pH5.0-5.5, molecular weight 60k, tetramer, GR, Nacalai Tesque Inc.) as a non-glycoprotein was used as a sample. We used four kinds of ion-exchangers (CM- Sephadex C-50, CM-Sepharose FastFlow, SP-Sephadex C-50, and SP-Sepharose FastFlow; all from Amersham Biosciences K.K.). Samples were added to the columns (0.1M Tris succinate buffer pH4.0) and washed (0.1M Tris succinate buffer pH4.0) according to the procedure in example 1. Elution was performed with a gradient of from ca pH4.0 to ca pH9.0, generated by a continuous decrease in the succinate concentration.
b. Result
   The results of chromatography with CM-Sephadex, CM-Sepharose, SP-Sephadex, and SP Sepharose are shown in Figs. 7-10, respectively.
   In each graph , the horizontal axis is the fraction number (time axis), and the spindle axis is OD at 280nm (left) and the pH of the elution solution (right).
   On CM-Sephadex (Fig. 7), the peak of ovalbumin is in the neighborhood of fraction number 26, the peak of streptavidin is in the neighborhood of fraction number 37, and the peak of transferrin is in the neighborhood of fraction number 39. On CM-Sepharose (Fig. 8), the peak of ovalbumin is in the neighborhood of fraction number 16, the peak of streptavidin is in the neighborhood of fraction number 35, and the peak of transferrin is in the neighborhood of fraction number 38 On SP-Sephadex (Fig. 9) the peak of ovalbumin is in the neighborhood of fraction number 13, the peak of streptavidin is in the neighborhood of fraction number 28, and the peak of transferrin is in the neighborhood of fraction number 37. On SP-Sepharose (Fig. 10), the peak of ovalbumin is in the neighborhood of fraction number 19, the peak of streptavidin is in the neighborhood of fraction number 31, and the peak of transferrin in the neighborhood of fraction number 38.

c. Evaluation
   Two kinds of glycoproteins and a non-glycoprotein (streptavidin) adsorbed to all of the ion-exchangers tested using Tris succinate buffer with a high ion strength, and were eluted in the vicinity of the isoelectric point. As shown in Figs. 9 and 10, individual glycoproteins could be separated with high accuracy even if the sample contained a plurality of glycoproteins. Under the conditions in this experiment streptavidin, a non-glycoprotein, was also seen to adsorb to the ion-exchanger. Furthermore, an N-type glycoprotein, which was used in the examination, showed high adsorption and excellent elution.
   This method shows excellent generality. It was demonstrated that the method of the invention is applicable to purification of a variety of glycoproteins.

### (3) Examination of the effect of the buffer concentration

In the experiment mentioned above (2), 0.1M Tris succinate buffer was used as a buffer solution for both adsorption and washing. An experiment similar to (2) was performed using 0.2M Tris succinate buffer (pH4.0) to examine the relation between the buffer concentration (ion strength) and the adsorption and elution characteristics.
a.Materials and methods
   While 0.2M Tris succinate buffer (pH4.0) was used for adsorption and washing, elution was performed with a gradient of from pH4.0 to pH9.0 with a continuous decrease in the succinate concentration.
   A solution that contained ovalbumin (isoelectric point pH4.5, molecular weight 45k, Sigma-Aldrich Corporation) and transferrin (isoelectric point pH6.2, molecular weight 80k, Sigma-Aldrich Corporation) as glycoproteins and streptavidin (isoelectric point of total protein pH6.4, isoelectric point of the surface pH5.0-5.5, molecular weight 60k, tetramer, GR, Nacalai Tesque Inc.) as a non-glycoprotein was used as a sample.
   CM-Sephadex was used as an ion-exchanger.
   Other experimental conditions were the same as in (2).
b. Result
   Figure 11 shows the results of chromatography. The horizontal axis is the fraction number (time axis) and the spindle axis is OD at 280nm. Ovalbumin shows a peak in the neighborhood of fraction number 16 and transferrin shows a peak in the neighborhood of fraction number 38.
   The non-glycoprotein streptavidin did not adsorb to the ion-exchanger.
c. Discussion
   While the non-glycoprotein streptavidin was not adsorbed when 0.2M Tris succinate buffer was used, both of the glycoproteins adsorbed to the ion-exchange material. Thus, a glycoprotein could selectively adsorbed to ion-exchanger by properly adjusting the buffer concentration. Thus, the simple purification method of the invention is effective for separating glycoproteins.
   On the other hand, although the non-glycoprotein also adsorbed to the ion-exchanger similar to a glycoprotein under the conditions used in experiment (2), glycoproteins showed a stronger adsorption tendency compared with the non-glycoprotein. Therefore, if the ion strength is set high enough so that a non-glycoprotein can not adsorb to the ion-exchanger, a glycoprotein can be adsorbed specifically. In other words, when adsorption is performed under the conditions that non-glycoproteins can not adsorb to the ion-exchanger, a target glycoprotein can be purified from a sample containing non-glycoproteins mixed together.

### (4) Effect of washing with a high concentration of 0.2M Tris succinate buffer

After adsorption under the same conditions as in (3), washing was performed using a large amount of buffer (about 200ml; 10 times or more the column capacity). Elution was then perfomed under the same conditions as in (3). The results are shown in Fig. 12.
Although the peak of transferrin in the neighborhood of fraction number 39 is somewhat smaller than that in Fig. 11, the peak of ovalbumin in the neighborhood of fraction number 20 is much smaller than that in Fig. 11.
Thus, glycoproteins that had adsorbed to the ion-exchanger continued to flow gradually upon washing for a long time with a high-concentration buffer. It was showed that the difference in adsorbability makes a difference in the effect of washing.
Considering the results of this experiment and those of (2) and (3), it is desirable to use as high an ion strength as possible when a target protein is adsorbed to the ion-exchanger. However, the outflow tendency increases when washing is performed under a high ion strength. Thus, a glycoprotein adsorbed under a high ion strength can be prevented from leaking out by washing under a low ion strength, and high-yield collection is possible.

### (5) Separation of a glycoprotein and a common protein with a weak bond

An experiment similar to (2) was performed using 20mM Tris succinate buffer (pH4.5) to examine the relation between the adsorption and elution characteristics of several proteins that were not adsorbed with 0.1M Tris succinate buffer used in (2) and glycoproteins that were not strongly adsorbed.
Carbonic anhydrase (Sigma c7025) and peroxidase (Wako Pure Chemicals 309-50993) were adsorbed to CM-Sephadex as a cation-exchanger using 20mM Tris succinate buffer (pH 4.5). Elution was performed with a linear gradient (from ca pH4.5 to ca pH8.5) by adding 20mM Tris after washing.
Figure 13 shows the results of chromatography. Fraction numbers 29-35 and 39-47 show the elution peaks of carbonic anhydrase and peroxidase, respectively.
Carbonic anhydrase and peroxidase did not adsorb to CM-Sephadex with 0.1M Tris succinate buffer (pH 4.5) (data not shown). However, when 20mM buffer was used in adsorption, it was possible to separate the peaks of carbonic anhydrase at pH 5.0 and peroxidase at pH 6.5.
The appearance of the peak of carbonic anhydrase is delayed when carbonic anhydrase and transferrin were adsorbed and washed with 20mM Tris succinate buffer (pH 4.5) and then eluted with a linear gradient in which 20mM Tris was added to the buffer (from ca pH4.5 to ca pH8.5), i.e., under a condition similar to that in the above experiment, and followed the peak of transferrin (not shown in the figure).
This method makes it possible to separate a glycoprotein and a common protein with an exceptionally weak bond under high ion strength because adsorption occurs under a low ion strength.
Thus, this method could be used to separate proteins which are not adsorbed onto the column under high ion strength (under a high buffer concentration), by adjusting the concentration etc. of the buffer.

### [Example 3]

### <Examination of the buffer composition and its relation to the adsorbent>

We examined how the adsorption of the glycoprotein to an ion-exchanger changed according to the kind of buffer. Tris acetate buffer and Tris citrate buffer were examined.
a. Materials and methods
   0.1M Tris acetate buffer (pH4.0) was used for adsorption and washing, and a gradient of from ca pH4.0 to ca pH 9.0, generated by a continuous decrease in the acetic acid concentration, was used for elution (condition 1).
   Tris citrate buffer (pH4.0) was also used for adsorption and washing, and a gradient o from ca pH4.0 to ca pH 9.0 generated by a continuous decrease in the citric acid concentration, was used for elution (condition 2). Ovalbumin, transferrin, and streptavidin were used for the sample. CM-Sephadex was used as an ion-exchanger.
b. Result
   The results of chromatography under conditions 1 and 2 are shown in Figs. 14 and 15, respectively. In each figure, the horizontal axis is the fraction number (time axis) and the spindle axis is OD at 280nm (left) and the pH of the eluate (right). In Fig. 14, the peak of all proteins applied to the column is in the vicinity of fraction number 32. Similarly, in Fig. 15, the peak of all proteins applied to the column is in the vicinity of fraction number 27.
c. Discussion
   Excellent adsorption and elution of glycoprotein were seen with both Tris acetate buffer and Tris citrate buffer. Thus, various buffers can be used in this method. Therefore, an appropriate buffer can be selected based on a consideration of the intended use of the target protein after extraction and the isoelectric point of the target glycoprotein, the isoelectric point of other coexisting glycoproteins and non-glycoproteins.

As shown in the above example, the method of the invention can be used to quickly and efficiently collect a target protein or glycoprotein from an incoherent sample that contains many kinds of protein and glycoprotein. Moreover, all of the glycoproteins that were subjected to examination could be collected, and in the glycoprotein, reversible adsorption was seen under a high ion strength. Therefore, this method shows high generality for the separation of glycoproteins.

### Industrial Applicability

The invention may be applied for various objects as the methods for separating and purifying proteins. For example, the invention is applicable for separation and recovery of useful proteins (disease-related glycoproteins) from biological samples (for example serum), and for detecting specified proteins (for example, a specified glycoprotein is compared between normal persons and patients to use the results for diagnosis). The invention may be used as a separation method when proteins or glycoproteins should be eliminated such as production of allergy-free vaccines. The invention may be also used for detecting or recognizing isoelectric points of various proteins. The invention is also useful for separating and purifying a target glycoprotein from a sample containing foreign proteins being different only in the presence or absence of sugar chains.
While the invention may be advantageous in separation and purification of the glycoprotein, the invention can be also applied for efficiently separating common proteins as well as various glycoproteins by combining the buffer solutions and columns.

The invention is by no means restricted to the above-mentioned aspects and examples of the invention. Instead, various modifications may be involved in the invention within a range capable of being readily presumed by those skilled in the art without departing from the scope as set forth in the appended claims.

## Claims

1. A method for separating proteins comprising the steps of:
(a) adsorbing a target protein on an ion exchanger by allowing a sample containing the target protein to contact the ion exchanger under a first condition at high ion strength and at a pH outside of the vicinity of the isoelectric point of the target protein; and
(b) eluting the component adsorbed on the ion exchanger under a second condition at lower ion strength than in the first condition, and at a pH closer to the isoelectric point side of the protein in the first condition.

2. The method for separating proteins according to Claim 1, wherein the first condition comprises using a buffer solution with a concentration of 0.05 M or more.

3. The method for separating proteins according to Claim 1, wherein the first condition comprises using a high concentration of the buffer solution comprising a combination of a weak acid and weak base.

4. A method for separating proteins comprising the steps of:
(a) adsorbing a target protein on an ion exchanger by allowing a sample containing the target protein to contact the ion exchanger under a first condition at first ion strength and at a pH outside of the vicinity of an isoelectric point of the target protein; and
(b) eluting the component adsorbed on the ion exchanger under a second condition at ion strength equal to or lower than in the first condition, and at a pH closer to the isoelectric point side of the protein in the first condition.

5. The method for separating proteins according to Claim 1 comprising the following step interposed between step (a) and step (b):
(c) washing the ion exchanger under a condition not eluting the target protein adsorbed on the ion exchanger.

6. The method for separating proteins according to Claim 5, wherein step (c) is applied under a substantially the same condition as in the first condition.

7. The method for separating proteins according to Claim 1, wherein
the pH in the first condition is lower than the isoelectric point of the target protein,
the ion exchanger is a cation exchanger, and
the pH in the second condition is in the vicinity of or higher than the isoelectric point of the target protein.

8. The method for separating proteins according to Claim 1, wherein
the pH in the first condition is higher than the isoelectric point of the target protein,
the ion exchanger is an anion exchanger, and
the pH in the second condition is in the vicinity of or lower than the pH corresponding to the isoelectric point of the target protein.

9. The method for separating proteins according to Claim 1, wherein the first condition comprises using a tris-succinate buffer.

10. The method for separating proteins according to Claim 1, wherein the second condition comprises using a buffer solution comprising a combination of the same acid and same base as in the buffer solution used in the first condition.

11. The method for separating proteins according to Claim 1, wherein the second condition comprises using a buffer solution having a pH in the vicinity of the isoelectric point of the target protein.

12. The method for separating proteins according to Claim 1, wherein
the sample contains a plurality of target proteins, and
step (b) comprises the step of continuously eluting the target proteins under a solvent condition corresponding to the isoelectric point of each protein.

13. The method for separating proteins according to Claim 1, wherein the protein is a glycoprotein.
